# NEUE EUROPÄISCHE PATENTSCHRIFT

(11) **EP 1 411 874 B2**
(45) Veröffentlichungstag und Bekanntmachung des Hinweises auf die Entscheidung über den Einspruch: **20.11.2019**
(45) Hinweis auf die Patenterteilung: 13.04.2016
(21) Anmeldenummer: 00977599.0
(22) Anmeldetag: 30.11.2000
(51) Int. Cl.: A61F 5/44, A61F 5/445

(54) **ABSORPTIONSKÖRPER ZUM ANSCHLUSS AN HAUT- UND SCHLEIMHAUTOBERFLÄCHEN**
ABSORBENT BODIES FOR APPLYING TO SKIN AND MUCUSA SURFACES
ELEMENT D'ABSORPTION A APPLIQUER SUR LA PEAU OU LES MUQUEUSES

(30) Priorität: 19.01.2000 DE 20000887 U
(43) Veröffentlichungstag der Anmeldung: 28.04.2004
(60) Teilanmeldung: 10003174.9
(73) Patentinhaber: BSN medical GmbH, 20253 Hamburg (DE)
(72) Erfinder: Riesinger, Birgit, 48149 Münster (DE)
(74) Vertreter: FARAGO Patentanwälte
(86) Internationale Anmeldenummer: PCT/EP2000/012007
(87) Internationale Veröffentlichungsnummer: WO 2001/052780

(56) Entgegenhaltungen:
- WO-A1-94/10956
- WO-A1-94/20054
- WO-A1-97/28773
- DE-C1- 19 752 598
- GB-A- 1 139 715
- US-A- 3 507 282
- US-A- 4 341 217
- US-A- 4 423 101
- US-A- 4 704 112
- US-A- 5 906 879
- US-A- 5 961 501
- US-A- 6 011 194

## Beschreibung

Die Erfindung betrifft einen Absorptionskörper zum Aufsaugen von flüssigen Ausscheidungen aus erkrankungsbedingten menschlichen Körperöffungen und -höhlen enthaltend eine Menge an Absorptionsmaterial,wie aufsaugendes Vlies das mit wenigstens einer flüssigkeitsdurchlässigen Abdeckschicht hinterlegt ist.

In DE-AS 19 62 331 ist ein hochsaugfähiger, faseriger Absorptiopskörper beschrieben, mit dem kollagenhaltige, textile Außenschichten fest verbunden sind. Die textile Außenschicht ist aus einem Fasergemisch aus Zellwolle und Zellulose, oder aus Synthesefasern hergestellt. Das entstandene Flachgebilde kann als Wundauflagematerial bzw. als Wundkissen verwendet werden, das direkt auf die Wunde aufzubringen ist. Nachteilig bei dem bekannten Absorptionskörper ist, daß er nur in der ersten Wundheilungsphase wirksam ist. Bei beginnender Epithelisation werden die Teilchen des neu gebildeten Hautgewebes mitgerissen, sobald das Wundkissen von der Wunde entfernt wird. Dies betrifft auch die aus Synthesefasern bestehende Abdeckung, die eine verbesserte Durchlässigkeit der Ausscheidungen gegenüber den Naturfasern aufweist. Das Wundkissen ist relativ teuer in Herstellung wegen Verfestigung und Verschweißung des Wundauflagematerials. Die Verwendung des beschriebenen Wundkissens ist zum Aufsaugen von flüssigen Ausscheidungen aus Wunden beschränkt.

Die WO9410953 offenbart Wundauflagen, die Superabsorber aufweisen können. Dies Schrift offenbart das Absorptionsmaterial ein aufsaugendes Vlies aufweist, damit einem Superabsorber-Granulat oder -Pulver durchsetzt ist, nicht jedoch das das Superabsorber-Granulat oder -Pulver ein Natriumacrylat Acrylsäure-Polymerisat aufweist.

Aufgabe der Erfindung ist, einen technisch vereinfachten und kostengünstigen Absorptionskörper zu konzipieren, der sich durch verbesserte Saugfähigkeit auszeichnet.

Diese Aufgabe ist durch einen Absorptionskörper nach Anspruch 1 gelöst.

Das Material der Hülle kann derart strukturiert sein, daß seine die Maschen begrenzenden Fäden- bzw. Faserabschnitte - im Schnitt durch die Hülle gesehen jeweils etwa bogenförmig sind und mit ihren Bogen-Scheiteln nach außen gerichtet sind. Diese Struktur hat sich als besonders vorteilhaft erwiesen, da zum ersten die aufzusaugenden Ausscheidungen schneller durch die Perforation bzw. durch die Maschen durchdringen können und zum zweiten die aktive Kontaktfläche der Hülle mit der Wunde gegenüber einer herkömmlichen, flachen Materialstruktur wesentlich verkleinert ist. Im günstigsten Fall kommen mit der Oberfläche der Wunde nur die Bogen-Scheitel in Kontakt. Dadurch läßt sich die Hülle auch einfacher von der Wunde entfernen. Zum dritten wird die vorgenannte räumliche Fixierung der Ausscheidungen innerhalb der Hülle dadurch unterstützt, dass die flüssigen Moleküle einen erschwerten Rücklauf haben, falls sie noch nicht gänzlich durch das Absorptionsmaterial aufgesaugt worden sind.

Zur Verbesserung der Saugfähigkeit des Absorptionskörpers trägt auch die Maschengrösse der Hülle 2 bei. Die Maschengrösse kann im Bereich 0,1 mm bis 10,0 mm, vorzugsweise 0,25 mm bis 1,2 mm liegen. Eine unterhalb dieses Bereichs liegende Maschengrösse wäre in einigen Fällen für den Transport der Ausscheidungen, insbesondere dickflüssigen, ungünstig.

Als Material der Hülle eignen sich insbesondere perforierte bzw. maschenartige Polyolefin-Folien, wie Polyethylen-oder Polypropylen-Folie, oder ein Naturstoff-Gewebe oder-vlies, das mit hautverträglichen Substanzen, wie Silicon, Paraffin, Wachs und dgl. imprägniert ist.

Der im wesentlichen flächenhafte Absorptionskörper kann oval, kreisrund, polygonal bzw. trapez-oder tropfenförmig sein. Es ist nicht ausgeschlossen, den Absorptionskörper in einer deutlich dreidimensionalen Form, wie Walze oder Kugel, herzustellen.

Diese Struktur des Materials mit bogenförmigen Fäden-oder Faserabschnitten kann auch bei einer abweichenden Auffangbeutel-Konstruktion ausgenutzt sein, bei der wenigstens eine aus dem Material bestehende Trennwand innerhalb des Auffangbeutels angeordnet ist.

Das innerhalb der Hülle angeordnete Absorptionsmaterial ist aus Zellstoffasern gebildet. Es ist auch vorgesehen, das Absorptionsmaterial mit einem Natriumacrylat Acrylsäure-Polymerisat durchsetzt ist.

Sowohl die Hülle, als auch das innerhalb der Hülle liegende Absorptionsmaterial kann mit einem geruchshemmenden und/oder - neutralisierenden bzw.-maskierenden Zusatz versehen sein, beispielweise mit Aktivkohlefilter oder mit Zusätzen, mit denen aktiv z.B. Schwefel-Wasserstoff-Verbindungen aus dem gasförmigen Milieu entfernt werden können. Als geruchshemmende können Naturstoffe gelten, bzw. Extrakte daraus, bioinerte Materialien sowie Kunststoffe, die eine bestimmte Elektronegativität aufweisen, wodurch eine Anlagerung und Bindung von Schwefel-Wasserstoff-Verbindungen erfolgen kann. Hierbei weisen die Kunststoffmoleküle bestimmte geometrische Formen auf, wie Prismen oder Spiralen.

Insgesamt ist das so ausgelegte Absorptionsmaterial, sowie die oben beschriebene Hülle in der Lage, ein inneres, wundheilungsfördendes Feuchtklima zu erzeugen.

Zwischen dem Absorptionsmaterial und der äußeren Hülle kann eine zusätzliche, innere Umhüllung aus einem Material, wie Baumwolle oder Zellstoff, angeordnet sein, dessen Saugfähigkeit gering bzw. kleiner als die des eigentlichen Absorptionsmaterials ist. Eine solche innere Umhüllung erschwert den direkten Kontakt zwischen den Schleimhautzellen an der Wunde oder Körperhöhle und dem Absorptionsmaterial. Anstelle der inneren Umhüllung kann zu demselben Zweck zwischen dem Absorptionsmaterial und der äußeren Hülle eine Zwischenlage in Form eines Materialabschnittes aus Baumwolle, Zellstoff oder aus Kunstfasern angeordnet sein. Der gesamte Absorptionskörper kann in einen auswechselbaren, grobmaschigen Netzbeutel eingelegt sein.

Das Absorptionsmaterial kann aus zwei oder mehr innerhalb der Hülle angeordneten, flach liegenden Lagen bestehen, die gleiche oder unterschiedliche Saugkraft aufweisen. Weiterhin kann zwischen den Lagen eine Innenwand angeordnet sein, die die Lagen voneinander vollständig oder teilweise trennt.

Die Figuren zeigen im einzelnen:
- Fig.1: einen flächenhaften Absorptionskörper in Draufsicht auf seine Flachseite;
- Figuren 2a und 2b: weitere Absorptionskörpers, ebenso in Draufsicht auf seine Flachseite
- Figuren 6 und 7: einen schematischen Schnitt durch einen Absorptionskörper mit jeweils zwei Lagen vom Absorptionsmaterial;
- Fig.8: einen schematischen Schnitt durch einen Absorptionskörper mit einem an der Hülle angebrachten Folienabschnitt;
- Fig. 9b: einen schematischen Schnitt durch einen Absorptionskörper mit einer Abziehfolie;
- Fig.10a: eine innere Umhüllung zwischen Absorptionsmaterial und Hülle, ebenso in einem schematischen Schnitt;
- Fig.10b: einen Absorptionskörper mit einer zusätzlichen Zwischenlage;

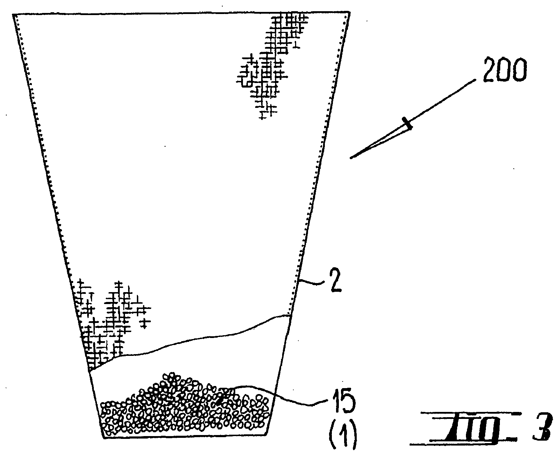

- Figuren 16 und 17: den auf die Haut des Patienten aufgelegten Absorptionskörper gemäß Figuren 1 bis 3;
- Figur 19: einen in einem Überzug angeordneten Absorptionskörper, aufgelegt auf die Wunde und dort befestigt;
- Figur 20: den erfindungsgemäßen, in einem Überzug angeordneten Absorptionskörper, wobei am Umfang des Überzuges einen Klebesseifen vorgesehen ist;
- Fig. 22: einen Material-Abschnitt der Hülle, in einer perspektivischen Sicht,
- Fig. 23: das Material der Hülle im Schnitt, in einer vergrösserten Darstellung

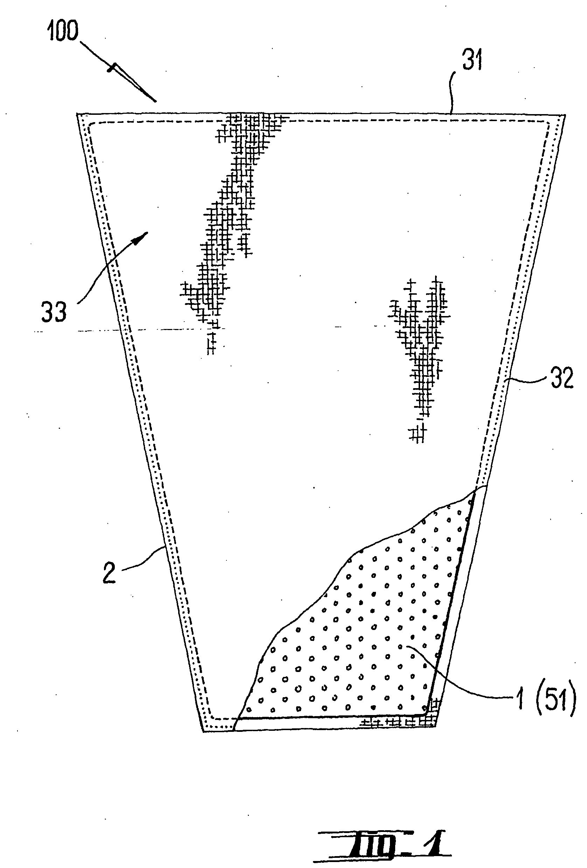

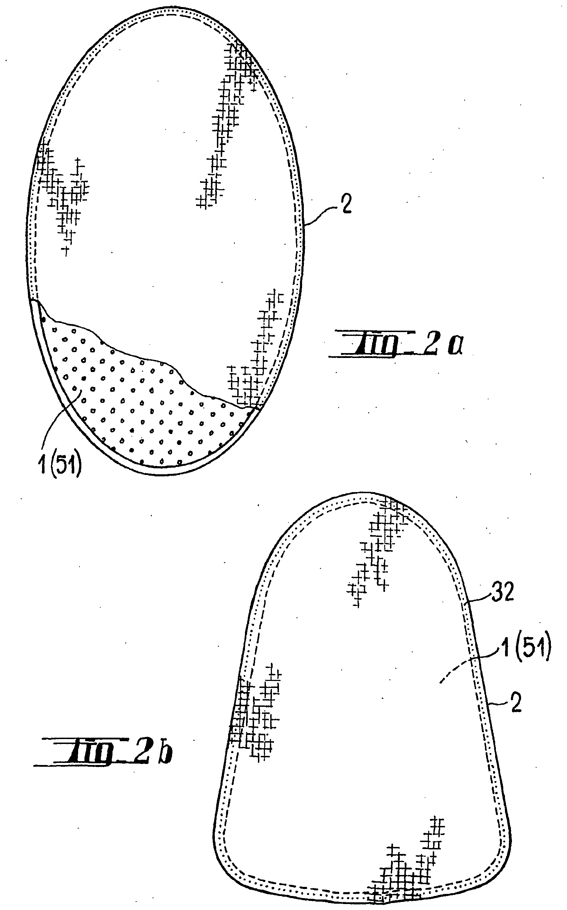

In Figuren 1 bis 2b ist ein Absorptionskörper in (Bezugszahl 100) dargestellt. Die Varianten des Absorptionskörpers 100 unterscheiden sich voneinander lediglich durch ihre äussere Form. In Fig. 1 ist ein trapezförmiger, in Fig. 2a ein ovaler und in Fig. 2b ein etwa tropfenförmiger Absorptionskörper gezeigt. Der Absorptionskörper 100 besteht aus einer Hülle 2 und einem innerhalb der Hülle 2 lose untergebrachten Absorptionsmaterial 1 in Form eines flachen Textil-Abschnittes 51.

Die Hülle 2 ist aus einem einstückigen, doppeltrapezförmigen Zuschnitt (nicht dargestellt) derart angefertigt, dass dieser um eine längs der Trapezbasis verlaufende Faltlinie 31 zusammengefaltet und an den übrigen Seitenkanten verschweisst (Naht 32) ist. Wie die Fig. 1 zeigt, ist der Textil-Abschnitt 51 ebenfalls trapezförmig. Der trapezförmige Absorptionskörper 100 weist ein Basismass von 11 cm und ein Mass an der Spitze von 5,5 cm mit einer Seitenlänge von 15 cm auf. Die Ausmasse können um einen Betrag von 2 cm variieren.

Die Hülle gemäss Figuren 2a und 2b besteht jeweils aus zwei gleichen, an ihrem Umfang miteinander verschweissten Seitenwänden.

Die Hülle 2 hat die Aufgabe, die Flüssigkeiten in das Innere des Absorptionskörpers zu transportieren. Die Hülle 2 bildet zugleich eine Barriere für feste, grobe Ausscheidungen.

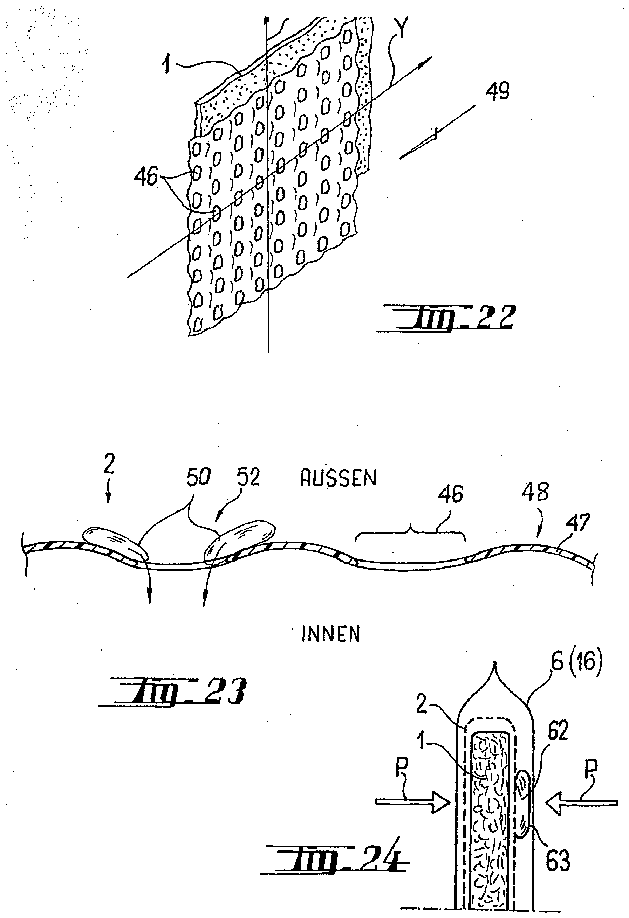

Die Figur 23 zeigt den Transportmechanismus von Flüssigkeiten. Die Hülle 2 besteht aus einer wasch-und desinfizierbaren, 30 jjm dicken Polyesterfolie mit einer Vielzahl von in beiden X, Y-Richtungen (vgl. Material-Abschnitt 49 ; Fig. 22) aufgereihten Maschen 46.

Wie den beiden Figuren 22,23 zu entnehmen ist, ist das Material der Hülle 2 derart strukturiert, dass seine die Maschen 46 begrenzenden Fäden-bzw. Faserabschnitte 47-im Schnitt durch die Hülle gesehen-jeweils etwa bogenförmig sind und mit ihren Bogen-Scheiteln 48 nach aussen gerichtet sind. Mit anderen Worten sind die Maschen bzw. Perforationen dem im Inneren der Hülle 2 platzieren Absorptionsmaterial 1 zugewandt und liegen jeweils in einer quasi Mulde 52 einer sich in beiden X, Y-Richtungen erstreckenden, etwa wellenförmigen Struktur.

Da das Material der Hülle 2 glatt und hydrophob ist, gleiten die Flüssigkeitsmoleküle 50 über die Aussenfläche der Hülle und treten einfacher durch die Perforationen ins Innere der Hülle 2 hindurch, wo sie von dem Absorptionsmaterial aufgenommen werden können. Erfahrungsgemäss ist die Maschengrösse zwischen 0,25 mm und 1,2 mm, im vorliegenden Fall etwa 0,5 mm optimal.

Selbstverständlich ist möglich, anstelle der beschriebenen eine andere Materialstruktur der Hülle zu wählen, beispielsweise eine wellenkammartige, bei der die Reihen von Perforationen jeweils in einer länglichen Mulde zwischen zwei Wellenkämmen liegen (nicht dargestellt).

Der in Figuren 1 bis 2b gezeigte Absorptionskörper 100 ist als Basisprodukt gedacht, das sich mit verschiedenen zusätzlichen Elementen, die noch im weiteren beschrieben werden, ergänzen oder ausstatten lässt.

Der innere Textil-Abschnitt 51 besteht aus einem mehrlagigen, hochsaugfähigen Zellstoff-Vlies von einer Dicke etwa 2 mm. Die Aussenschichten des Zellstoff-Vlieses sind ebenfalls perforiert, so dass die aufzusaugenden Flüssigkeitsmoleküle nicht nur aufgrund der Kapillarwirkung, sondern auch durch diese Teil-Perforationen in dessen Kern gelangen.

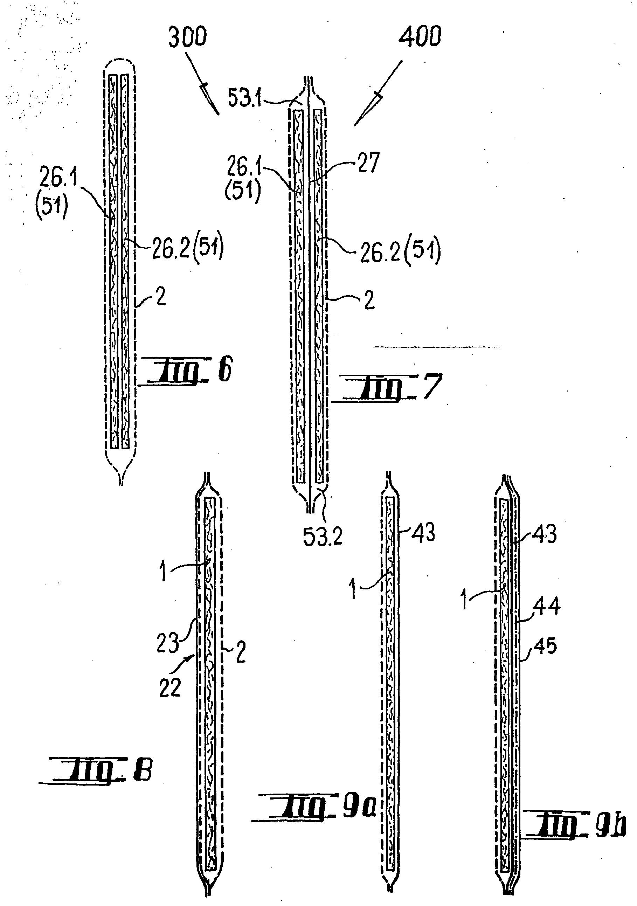

Die Figuren 6 und 7 zeigen weitere Absorptionskörper (Bezugszahlen 300 und 400). Als Absorptionsmaterial sind jeweils zwei Lagen 26.1,26.2 des bereits beschriebenen Textil-Abschnittes 51 vorgesehen, wobei bei der Ausführung gemäss Fig. 7 zwischen den Lagen 26.1,26.2 eine flüssigkeitsundurchlässige Innenwand 27 angeordnet ist, die den Absorptionskörper 400 in zwei gleiche Kammern 53.1, 53.2 unterteilt. Anstelle der flüssigkeitsundurchlässigen Innenwand 27 kann eine flüssigkeits- und/oder gasdurchlässige Membran (nicht dargestellt) Verwendung finden.

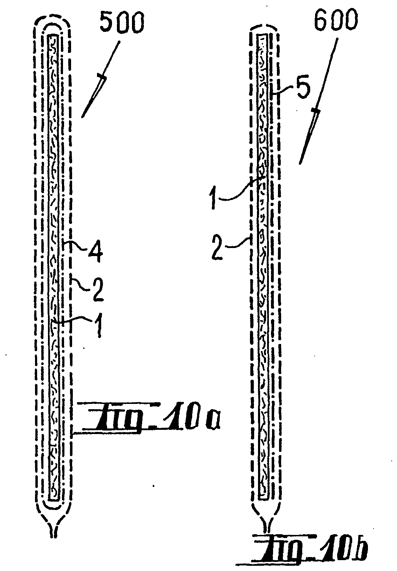

Bei einem in Fig. 10a gezeigten Absorptionskörper 500 ist zwischen der Hülle 2 und dem Absorptionsmaterial 1 eine weitere, innere, vollständige Umhüllung 4 aus Baumwolle angeordnet, deren Saugfähigkeit kleiner als die des eigentlichen, inneren Absorptionsmaterials ist. Die Umhüllung 4 erschwert den direkten Kontakt zwischen den Schleimhautzellen an der Wunde oder Körperhöhle und dem Absorptionsmaterial.

Bei einen in Fig. 10b dargestellten Absorptionskörper (Bezugszahl 600) ist anstelle der Umhüllung 4 eine Zwischenlage 5 in Form eines Materialabschnittes vorgesehen, die zwischen dem Absorptionsmaterial 1 und der Hülle 2 angeordnet ist. Die Zwischenlage 5 besteht ebenfalls aus Baumwolle und hat dieselbe Aufgabe, wie die Umhüllung 4.

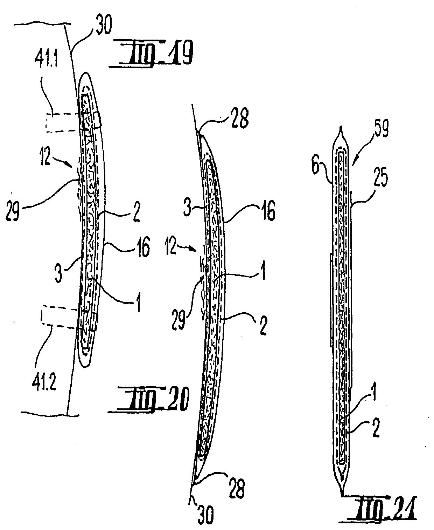

Der den Absorptionskörper umgebende Überzug 16 wird in Behandlung von Wunden verwendet. So zeigt die Fig. 19 einen mittels zwei Klebebänder 41.1,41.2 an der Haut 30 des Patienten Überzug 16, der jedoch eine körpernahe Öffnung 12 aufweist, damit die flüssigen, aus der Wunde 29 austretenden Substanzen direkt von dem innerhalb des Überzuges platzierten Absorptionskörper aufgesaugt werden können.

Wie die Fig. 20 zeigt, ist am Umfang des Überzuges 16 ein den Wundbereich abdichtendes Klebestreifen 28 vorgesehen.

Der aus der Hülle 2 und dem Absorptionsmaterial 1 bestehende Absorptionskörper ist zur direkten Behandlung von Wunden geeignet, ohne einen zusätzlichen Überzug verwenden zu müssen. Dieser kann mit Klebestreifen oder Gurten oder, wie die Figuren 16 und 17 zeigen, mit einem an seinem Umfang 21 abgebrachten Klebeband 61 abdichtend an der Haut 30 des Patienten befestigt sein. In Fig. 16 ist ein trapezförmiger und in Fig. 17 im Teilschnitt ein ovaler Absorptionskörper 100 gezeigt.

In den Figuren 8 bis 9b sind drei von dem Absorptionskörper abweichenden Absorptionkörper dargestellt. Gemäss Fig. 8 ist an einer äusseren Flachseite 22 der Hülle 2 ganzflächig ein flüssigkeitsundurchlässiger Folienabschnitt 23 angebracht. Der Folienabschnitt 23 ist mit der Hülle 2 an ihrem Umfang verschweisst.

## Patentansprüche

1. Absorptionskörper (100) zum Aufsaugen von flüssigen Ausscheidungen aus Wunden, enthaltend eine Menge an Absorptionsmaterial (1) aufweisend ein aufsaugendes Vlies, das mit wenigstens einer flüssigkeitsdurchlässigen Abdeckung, hinterlegt ist, wobei die Abdeckung einer Hülle entspricht, wobei das Absorptionsmaterial von der Hülle umgeben ist,
die aus einem uni- oder bidirektional flüssigkeitsdurchlässigen, schleimhautverträglichen bzw. hautverträglichen Natur- oder Kunststoff besteht,
an dem die Ausscheidungen nicht oder kaum haften und der die Flüssigkeiten durch sich durchtreten lässt und eine Barriere für feste grobe Ausscheidungen bildet und die Aufnahme bzw. das Aufsaugen von austretenden und/oder entfernungspflichtigen Substanzen durch das innerhalb der Hülle (2) angeordnete Absorptionsmaterial (1) ermöglicht,
so dass der direkte Kontakt der Ausscheidungen mit der Haut bzw. Schleimhaut im angeschlossenen Zustand des Absorptionskörpers (100) an die Körperöffnung oder - höhle eingeschränkt ist,
und die ausfließenden Ausscheidungen durch die Hülle (2) hindurchtreten, in das Absorptionsmaterial (1) gelangen und innerhalb der Hülle (2) räumlich fixiert verbleiben, wobei das Absorptionsmaterial (1) mit einem Superabsorber-Granulat oder -Pulver aufweisend ein Natriumacrylat Acrylsäure-Polymerisat durchsetzt ist, **dadurch gekennzeichnet, dass** der Absorptionskörper von einem Überzug (16) umgeben ist, der
• eine körpernahe Öffnung (12) und
• am Umfang des Überzuges (16) einen den Wundbereich abdichtenden Klebestreifen (28) aufweist,
und die flüssigen aus der Wunde austretenden Substanzen direkt von dem innerhalb des Überzugs platzierten Absorptionskörper aufgesaugt werden können.

2. Absorptionskörper nach Anspruch 1, **dadurch gekennzeichnet, dass** der Überzug (16) flächenhaft und flüssigkeitsundurchlässig ist.

3. Absorptionskörper nach Anspruch 1, **dadurch gekennzeichnet, dass** das Material der Hülle (2) eine perforierte bzw. maschenartige Polyolefin-Folie aufweist.

4. Absorptionskörper nach Anspruch 1, **dadurch gekennzeichnet, dass** das Material der Hülle (2) eine ein- oder mehrlagige, perforierte bzw. porenartige Polyolefin Folie, ist, oder aus einem Naturstoff-Gewebe oder -vlies hergestellt ist, das mit hautverträglichen Substanzen, wie Silicon, Paraffin, Wachs, imprägniert ist,
und dass das Material der Hülle (2) derart strukturiert ist, dass Fäden- bzw. Faserabschnitte (47), welche in der Hülle vorkommende Maschen (46) begrenzen - im Schnitt durch die Hülle gesehen-jeweils etwa bogenförmig sind und mit ihren Bogen-Scheiteln (48) nach außen gerichtet sind.

5. Absorptionskörper nach Anspruch 4, **dadurch gekennzeichnet, dass** die Maschen (46) oder Perforationen 0,10 mm bis 3 mm, vorzugsweise 0,25 mm bis 1,0 mm gross sind.

6. Absorptionskörper nach einem der Ansprüche 1 bis 5, **dadurch gekennzeichnet, dass** dieser flächenhaft ist.

7. Absorptionskörper nach einem der Ansprüche 1 bis 6, **dadurch gekennzeichnet, dass** das Absorptionsmaterial (1) im Wesentlichen aus Zellstofffasern gebildet ist.

8. Absorptionskörper nach einem der vorhergehenden Ansprüche, **dadurch gekennzeichnet, dass** zwischen dem Absorptionsmaterial und der äußeren Hülle (2) eine zusätzliche, innere Zwischenlage (5) in Form eines Materialabschnittes aus Baumwolle, Zellstoff oder dergleichen angeordnet ist,
wodurch der direkte Kontakt zwischen den Schleimhautzellen an der Wunde oder Körperhöhle und dem Absorptionsmaterial erschwert ist.

9. Absorptionskörper nach einem der vorhergehenden Ansprüche, **dadurch gekennzeichnet, dass** dieser bzw. wenigstens ein Teil des Absorptionskörpers sterilisiert oder sterilisierbar ist.

## Claims

1. Absorbent body (100) for absorption of fluid exudates from wounds, comprising an amount of absorption material (1) comprising an absorbing fleece, which is coated with at least one fluid permeable cover, wherein the cover represents a sheath, wherein the absorption material is wrapped by the sheath,
which is composed of a mono- or bidirectionally fluid permeable, mucosa friendly or skin friendly natural or synthetic material, respectively,
to which the exudates do not or hardly adhere and which is permeable for the fluids and forms a barrier for solid crude secretions and allows for the intake or absorption, respectively, of leaking substances and/or substances to be removed by the absorption material (1) disposed within the sheath (2),
so that the direct contact of the exudates with the skin or mucosa, respectively, is limited when the absorbent body (100) is connected to the body orifices or cavities, and the leaking exudates permeate through the sheath (2) into the absorption material (1) and remain locally retained within the sheath (2), wherein the absorption material (1) is interspersed with a superabsorber granulate or -powder comprising a sodium acrylate acrylic acid polymerizate,
**characterized in that** the absorbent body is surrounded by an overlay (16) comprising
• a body proximal opening (12) and
• an adhesive strip (28) sealing the wound area in the periphery of the overlay (16) and the fluid substances leaking from the wound can be directly absorbed from the absorption body placed within the sheath.

2. Absorbent body according to claim 1 **characterized in that** the overlay (16) is impermeable for fluids.

3. Absorbent body according to claim 1 **characterized in that** the material of the sheath (2) is comprising at least partially a perforated or mesh-like polyolefin film, respectively.

4. Absorbent body according to claim 1 **characterized in that**
the material of the sheath (2) is a mono- or multi-layered, perforated or pore-like polyolefin film, or composed of a natural tissue or -fleece, which is impregnated with skin-friendly substances such as silicone, paraffin or wax,
and that the material of the sheath (2) is structured so that thread or fiber areas (47) delimiting the meshes (46) contained within the sheath are - viewed in the section through the sheath - each about arched and directed outward with their arch crowns (48).

5. Absorbent body according to claim 4 **characterized in that** the meshes (46) or perforations are 0.10 mm to 3 mm, preferably 0.25 mm to 1.0 mm in size.

6. Absorbent body according to any of claims 1 - 5 **characterized in that** the same is laminar.

7. Absorbent body according to any of claims 1 - 6 **characterized in that** the absorption material (1) is essentially composed of pulp fibers.

8. Absorbent body according to any of the preceding claims **characterized in that** there is an additional inner intermediary layer (5) in the form of a stretch of material composed of cotton, pulp or alike, disposed between the absorption material and the outer sheath (2),
whereby the immediate contact between the cells of the mucosa at the wound or body cavity and the absorption material is hindered.

9. Absorbent body according to any of the preceding claims **characterized in that** the absorbent body or at least part of the absorbent body is sterilized or sterilizable.

## Revendications

1. Corps absorbant (100) pour l'aspiration d'excrétions liquides dans les plaies,
contenant une quantité de matériau absorbant (1) présentant un non-tissé absorbant qui est doublé d'au moins un recouvrement perméable à du liquide, le recouvrement correspondant à une enveloppe, le matériau absorbant étant entouré par l'enveloppe, qui consiste en un matériau naturel ou artificiel perméable à du liquide dans une ou deux directions et toléré par les muqueuses ou toléré par la peau,
auquel les excrétions n'adhèrent pas ou à peine et qui laisse les liquides passer à travers lui et qui constitue une barrière pour des excrétions grossières solides et qui permet la récupération ou l'absorption de substances sortantes et/ou devant être enlevées via le matériau absorbant (1) disposé dans l'enveloppe (2),
de sorte que le contact direct des excrétions avec la peau ou la muqueuse, en état raccordé du corps absorbant (100), est restreint à l'orifice ou à la cavité corporel(le), et que les excrétions qui s'écoulent traversent l'enveloppe (2), arrivent dans le matériau absorbant (1) et restent spatialement fixes dans l'enveloppe (2), le matériau absorbant (1) étant rempli de granulés ou de poudre super-absorbant(e)s contenant un polymérisât d'acrylate de sodium et d'acide acrylique,
**caractérisé en ce que** le corps absorbant est entouré d'un revêtement (16) qui présente
• une ouverture (12) proche du corps et,
• sur la périphérie du revêtement (16), une bande adhésive (28) isolant la zone de la plaie,
et les substances liquides d'une plaie suitante peuvent être absorbées directement par le corps absorbant placé à l'intérieur de l'enveloppe.

2. Corps absorbant selon la revendication 1, **caractérisé en ce que** le revêtement (16) est imperméable à du liquide.

3. Corps absorbant selon la revendication 1, **caractérisé en ce que** le matériau de l'enveloppe (2) présente du moins par endroits un film de polyoléfine perforé ou de type maille.

4. Corps absorbant selon la revendication 1, **caractérisé en ce que** le matériau de l'enveloppe (2) est un film de polyoléfine à une ou plusieurs couche(s), perforé ou poreux, ou est fabriqué à partir d'un tissu ou non-tissé en matériau naturel qui est imprégné de substances tolérées par la peau telles que du silicone, de la paraffine, de la cire, et que le matériau de l'enveloppe (2) est structuré de manière telle que des sections (47) de fils ou de fibres, qui délimitent des mailles (46) présentes dans l'enveloppe - vues en coupe à travers l'enveloppe - soient respectivement approximativement de forme arquée et soient dirigées vers l'extérieur par les sommets de leurs feuilles (48).

5. Corps absorbant selon la revendication 4, **caractérisé en ce que** les mailles (46) ou perforations ont une dimension de 0,10 mm à 3 mm, de préférence 0,25 mm à 1,0 mm.

6. Corps absorbant selon une des revendications 1 à 5, **caractérisé en ce que** celui-ci est en surface.

7. Corps absorbant selon une des revendications 1 à 6, **caractérisé en ce que** le matériau absorbant (1) est composé substantiellement de fibres de cellulose.

8. Corps absorbant selon une des revendications précédentes, **caractérisé en ce que**, entre le matériau absorbant et l'enveloppe extérieure (2), une couche intermédiaire intérieure supplémentaire (5) se présentant sous la forme d'une section de matériau en coton, cellulose ou similaire est disposée, ce qui rend plus difficile le contact direct entre les cellules des muqueuses à la plaie ou la cavité corporelle et le matériau absorbant.

9. Corps absorbant selon une des revendications précédentes, **caractérisé en ce que** celui-ci ou du moins une partie du corps absorbant est stérilisée ou peut être stérilisée.
